# EUROPEAN PATENT SPECIFICATION

(11) **EP 1 382 572 B1**
(45) Date of publication and mention of the grant of the patent: **23.08.2006**
(21) Application number: 03007518.8
(22) Date of filing: 01.04.2003
(51) Int. Cl.: C02F 1/32, A61L 2/10, A61L 9/20, A23L 3/28

(54) **Ultraviolet-radiation sterilizing fluid apparatus**
UV-Sterilisationsvorrichtung
Appareil de stérilisation à rayons UV

(30) Priority: 17.07.2002 CN 02125522; 16.12.2002 US 319464
(43) Date of publication of application: 21.01.2004
(73) Proprietor: Vast Light Ltd., Kowloon (HK)
(72) Inventor: Wong, Chi-Kin Tommy, Kowloon (HK)
(74) Representative: Haft, von Puttkamer, Berngruber, Czybulka

(56) References cited:
- EP-A- 0 202 820
- WO-A-02/38191
- WO-A-03/031338
- US-A- 5 384 032
- US-A- 5 853 572

## Description

### BACKGROUND OF THE INVENTION

### (a) Field of the invention

The present invention relates to an ultraviolet-radiation sterilizing fluid processor, especially to a turbine-boosted, ultraviolet-radiation sterilizing fluid processor which makes the fluid flow inside form a whirlpool spinning at a high speed so as to improve the sterilization effect.

### (b) Description of the Prior Art

A regular ultraviolet-radiation lamp processor involves an ultraviolet-radiation lamp installed inside a quartz sleeve. The flow rate of the fluid through the tube unit depends on the total energy of the ultraviolet radiation emitted from the lamp. When a fluid flows through the tube unit, the germs, algae or other organisms absorb the ultraviolet-radiation energy until the sterilizing effect is fatal. In a prior art of the tube unit, there is only a tube through which fluid flows. The fluid flow directly passes the interior, no matter if the ultraviolet radiation is emitted from the inside or outside to perform the sterilizing effects. Due to the restriction of the fluid flow rate, the slow-flowing fluid inside the tube unit is not blended properly, resulting in inconsistent exposure time while the fluid is being processed. Thus the sterilization is unsatisfactory. Furthermore, when the fluid passes by the tube unit, the cross-sectional area of passage is larger than the cross-sectional area of the fluid inlet tube, so the flow is slowed, resulting in accumulation of dirt onto the wall of the quartz sleeve, which obstructs and reduces the penetration of the ultraviolet radiation, and directly reduces the total energy of ultraviolet radiation absorbed by the organisms in the fluid. If all or part of the organisms in the fluid can not absorb a sufficient amount of ultraviolet energy to reach the fatal dosage when the fluid passes through the tube unit, there will be a total failure of the sterilizing functions. The loss of this sterilization is a major detriment.

Effects of sterilization are dependent on whether the ultraviolet energy can be absorbed sufficiently to reach to the fatal dosage of each living organism in the fluid. The effectiveness is dependent on whether the fluid is properly stirred and the degree of penetration of ultraviolet radiation. Therefore, the three major factors effecting the ultraviolet sterilization are: "consistent radiating time", "the penetration of the ultraviolet radiation" and the "absorption of a fatal dosage".

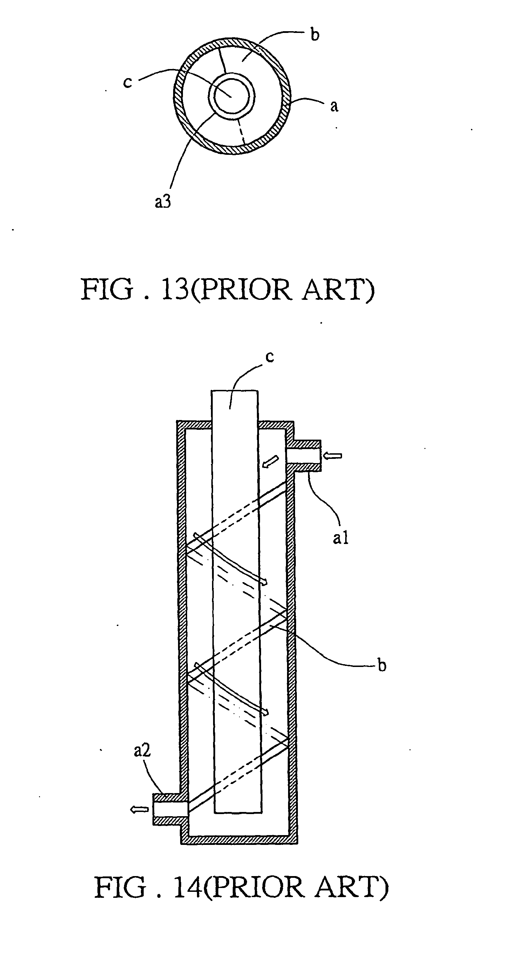

In analyzing the prior art of ultraviolet-radiation lamp processors it is found that they are deficient in the factors above. The construction of the present invention is shown in FIGS. 13 and 14, wherein inside a tubular casing (a) is a spiral diversion plate (b), on the upper and lower part of the tubular casing (a) is respectively a fluid inlet tube (a1) and a fluid outlet tube (a2), wherein fluid can flow into the fluid inlet tube (a1) and out of the fluid outlet tube (a2). Inside the tubular casing (a) is a hole through the center (a3) in which the ultraviolet lamp (c) is installed to achieve sterilizing objectives.

A common characteristic of other types of ultraviolet-radiation lamp processors disclosed in other patents, such as U.S. patent Nos. 5069885, 5785845, 5675153, 5605400, 1175948, 1822006 and 3754658, and Japanese Patent Nos. Zhao-59-150589 and Te-Kai-Zhao-57-75113, relates to a spiral conduction plate installed in a tube unit, so that fluid flow will rotate inside the tube unit, and the fluid will then be blended to achieve the consistent absorption of a fatal dosage.

However, in the above units the fluid flows through the spiral diversion plate (b) and spins around the ultraviolet-radiation lamp (c) but the line and speed of the fluid flow are restricted by the length of the conduction current plate, thus resulting in an insufficient blending of the fluid. In addition, the volume of flow and speed of the fluid flowing through each diversion channel are not consistent and the desired sterilizing effect will not be achieved.

Moreover, the spiral diversion plate (b) only provides guidance to lead the fluid to flow at a low speed, and the extraneous matter contained in the fluid will easily accumulate on the surface of the ultraviolet-radiation lamp (c) thus obstructing and reducing the penetration of ultraviolet radiation, and reducing the sterilizing effects of the ultraviolet-radiation lamp (c).

Additionally, the tubular casing (a) has a spiral diversion plate (b) that is quite sophisticated in its configuration, causing increased production costs and reduced market competitiveness.

The document WO 02/38191 A2 describes a method of inactivating mircroorganisms in a fluid using ultraviolet radiation. In this document a UV reactor is described which includes an elongated UV lamp disposed within a quartz tube. The quartz tube is surrounded by a housing which defines a reaction chamber having a fluid inlet port and a fluid outlet port. The inner wall of the housing is machined with an array of annular chambers separated by respective partitions which extend towards but do not engage the quartz tube to define relatively narrow passages between the partitions and the quartz tube. These narrow passages allow the fluid to move from the inlet port to the outlet port while being treated by the UV lamp.

Document U.S. 5,384,032 describes a water purifying and sterilizing apparatus including a box with three filtering chambers, one of which has an ultraviolet lamp and is provided with a flow baffle comprised of a circular part centrally provided with a circular orifice that surround the said lamp, being made up of U-shaped sections, interconnected by ramps describing rectangular openings for water to flow through.

Another home water purification system is described in the U.S. 5,853,572 comprising a source of radiant ultraviolet energy having an elongate ultraviolet discharge lamp having an elongate central axis defining an axial direction, means for establishing plug like spiral flow with a substantial axial and tangential component about that elongate ultraviolet discharge lamp between a source water inlet and a source water outlet.

According to these constructions the fluid pressure inside the devices is the fluid pressure provided by the water inlet.

This means that if the flow rate of the fluid is restricted, no sufficient blending of the fluid can take place in order to achieve a good sterilization effect.

### SUMMARY OF THE INVENTION

The main objective of the present invention is to provide a turbine-boosted, ultraviolet-radiation sterilizing fluid processor with a diversion mechanism on top of a tube unit, by which the fluid flowing into the unit forms a whirlpool rotating at a high speed even if the flow rate of fluid is restricted, enabling sufficient blending of the processed fluid to achieve maximum sterilization effect.

In order to achieve the objective mentioned above and avoid the shortcomings of the prior art, the present invention includes a quartz sleeve installed inside an outer tube unit, inside which is installed an ultraviolet-radiation lamp. At the upper and lower end of the tube unit is respectively a fluid inlet tube and a fluid outlet tube.

A diversion mechanism is disposed on top of the tube unit and under the fluid inlet tube respectively. The diversion mechanism is composed of a plurality of diversion channels arranged in a ring unit. The total area of the fluid outlet hole of the diversion channel is smaller than the cross-sectional area of the passage of the tube unit so as to form a fluid-pressurizing chamber on top of the tube unit. Thus the fluid flowing through is pressurized and therefore blended properly.

The diversion mechanism of the present invention includes a baffle disposed below a ring unit and a plurality of diversion channels in the shape of round holes on top of the baffle.

The holey diversion channels are inclined at a specified angle. The fluid inlet hole of the diversion channel is larger than the fluid outlet hole of the diversion channel.

The diversion channel of the present invention is formed between the two adjacent spiral blades arranged on the diversion mechanism.

The inclination angle of the spiral blade of the present invention ranges from 91 to 179 degrees.

The downward inclination angle of the tapered protrusion located at the lower part of the diversion mechanism of the present invention ranges from 1 to 89 degrees.

The upward inclination angle of the tapered depression located at the lower part of the diversion mechanism of the present invention ranges from 1 to 89 degrees.

The fluid inlet hole of the diversion mechanism of the present invention is located at the upper part of the diversion channel, while the fluid outlet hole is located at the lower part of the diversion channel.

The diversion mechanism of the present invention is a ring unit, having a fluid inlet hole located on an outside rim of the diversion channel, and a fluid outlet hole on an inside rim of the diversion channel.

The diversion mechanism of the present invention is a ring unit, having a fluid inlet hole located on an inside rim of the diversion channel, and a fluid outlet hole on an outside rim of the diversion channel.

By the present invention, a whirlpool spinning at a high speed is formed inside the tube unit no matter whether the flow rate of the fluid is low or high, so that the fluid for the sterilizing function can be blended properly, thus increasing its sterilizing effects. Also the effect of the fluid spinning at high speed against the wall of the quartz sleeve reduces the accumulation of dirt on the wall of the quartz sleeve, thus reducing the need for frequent maintenance.

### BRIEF DESCRIPTION OF THE DRAWINGS

The accomplishment of the above-mentioned object of the present invention will become apparent from the following description and its accompanying drawings, which illustrate the embodiment of the present invention, and are as follows:
FIG. 1 is an exploded view of the invention.
FIG. 2 A & B are cross-sectional views of the invention when assembled.
FIG. 3 is a perspective top view of the diversion mechanism having a plurality of spiral blades in the invention.
FIG. 4 is a sectional view of the diversion mechanism in FIG. 3.
FIG. 5 is a cross-sectional view of another embodiment of the invention.
FIG. 6 is a perspective top view of the diversion mechanism in FIG. 5.
FIG. 7 is a perspective bottom view of the diversion mechanism in FIG. 5.
FIG. 8 is a cross-sectional view of a further embodiment of the invention.
FIG. 9 is a perspective top view of the diversion mechanism in FIG. 8.
FIG. 10 is a perspective bottom view of the diversion mechanism in FIG. 8.
FIG. 11 is a cross-sectional view of a further embodiment of the invention.
FIGS. 12A and 12B are transverse cross-sectional views of another embodiment of the diversion mechanism in the invention.
FIG. 13 is a cross-sectional view of a prior art.
FIG. 14 is a top section view of a prior art.

### DETAILED DESCRIPTION OF THE PREFERRED EMBODIMENT

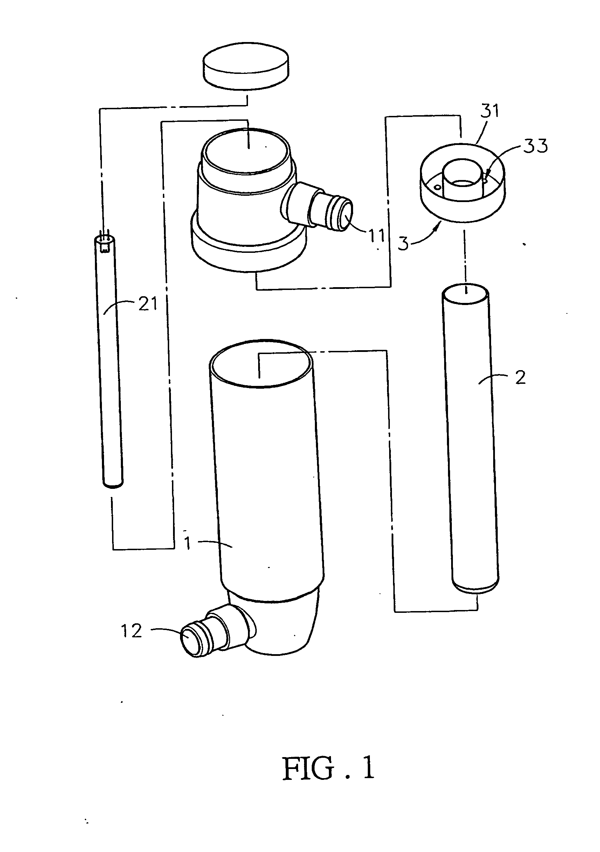

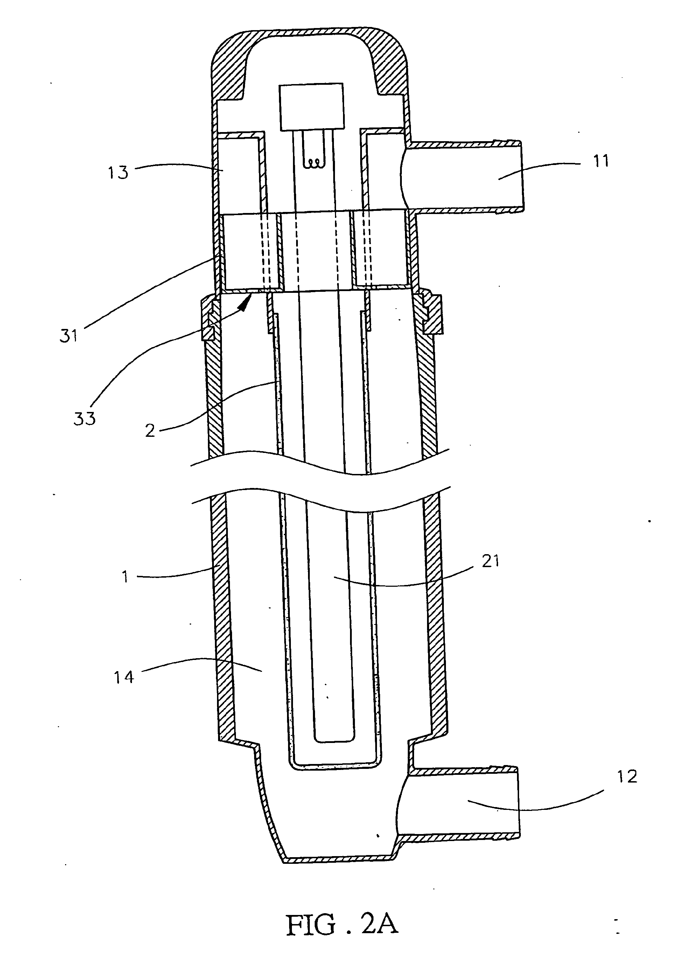

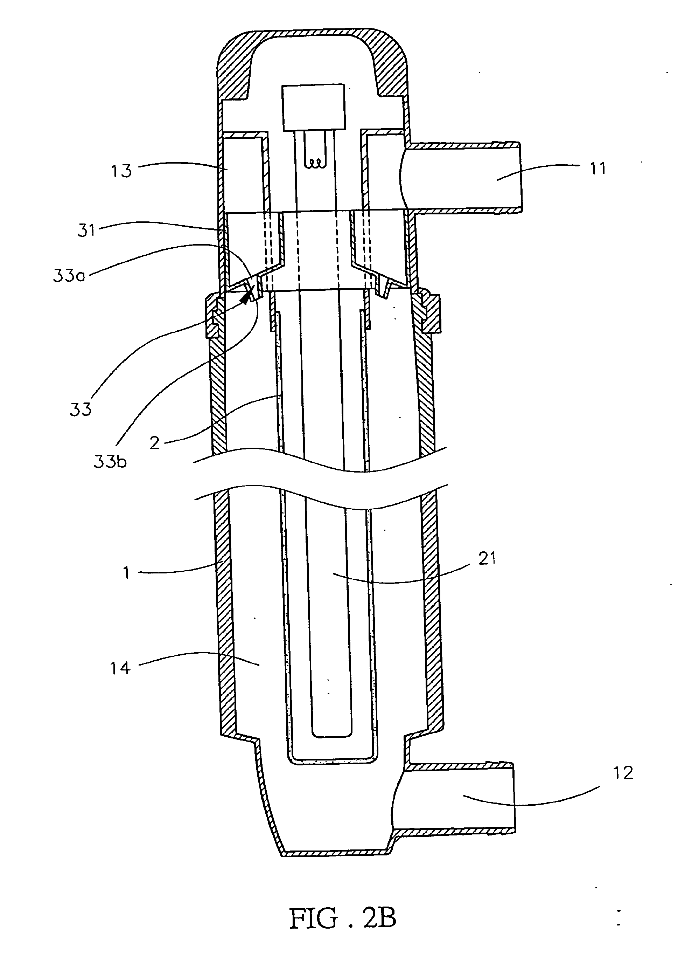

As shown in FIGS. 1 and 2, the invention comprises a quartz sleeve 2 installed inside a tube unit 1, wherein is installed an ultraviolet-radiation lamp 21, and on the upper and lower ends of the tube unit 1 is a fluid inlet tube 11 and a fluid outlet tube 12; the invention is characterized in that:
Inside the tube unit 1 and below the fluid inlet tube 11 is installed a diversion mechanism 3,
The diversion mechanism 3 includes a plurality of diversion channels 33 disposed in a ring unit 31. The total area of the fluid outlet hole of the diversion channel 33 is smaller than the cross-sectional area of the passage 14 of said tube unit 1 (refer to FIG. 2) so as to form a fluid-pressurizing chamber 13.

Refer to embodiments of the present invention shown in FIG. 1, FIG. 2 A&B, a diversion mechanism 3 having a baffle 31a disposed below a ring unit 31 and a plurality of holey diversion channels 33 (see FIG. 2A) on top of the baffle 31a. Or refer to FIG. 2B, the diversion mechanism 3 having the baffle 31a disposed below the ring unit 31 and a plurality of inclined diversion channels 33 on top of the baffle 31a. The fluid inlet hole 33a of the diversion channel 33 is larger than the fluid outlet hole 33b of the diversion channel 33. Moreover, the baffle 31a can be designed into an inclined plane or a camber protruding upwardly or depressed downwardly.

In accordance with the structure mentioned above, fluid flows into the tube unit 1 through the fluid inlet tube 11, since the total sectional area of the diversion channel 33 of the diversion mechanism 3 is getting smaller so the fluid flowing through the passage 14 is pressured and accelerated for being blended properly. Therefore, the organisms inside the fluid can be radiated in "consistent radiating time" and also has "absorption of a fatal dosage" so as to increase effects of sterilization.

Moreover, the fluid is pressured through the regulation of the diversion mechanism 3 and is flowing into the passage 14, creating a whirlpool spinning at a higher speed. The accelerated whirlpool can flush the surface of the surface of the quartz sleeve 2 so that dirt can't accumulate on the surface of the quartz sleeve 2 of the ultraviolet-radiation lamp 21. Hence "the penetration of the ultraviolet radiation" is increased. Also the sterilizing effects are increased and the frequency for maintenance is reduced.

In addition, the structure of the diversion mechanism 3, compared to the prior art with a plurality of spiral guiding plates, is simplified, thereby reducing production cost and increasing its market competitiveness.

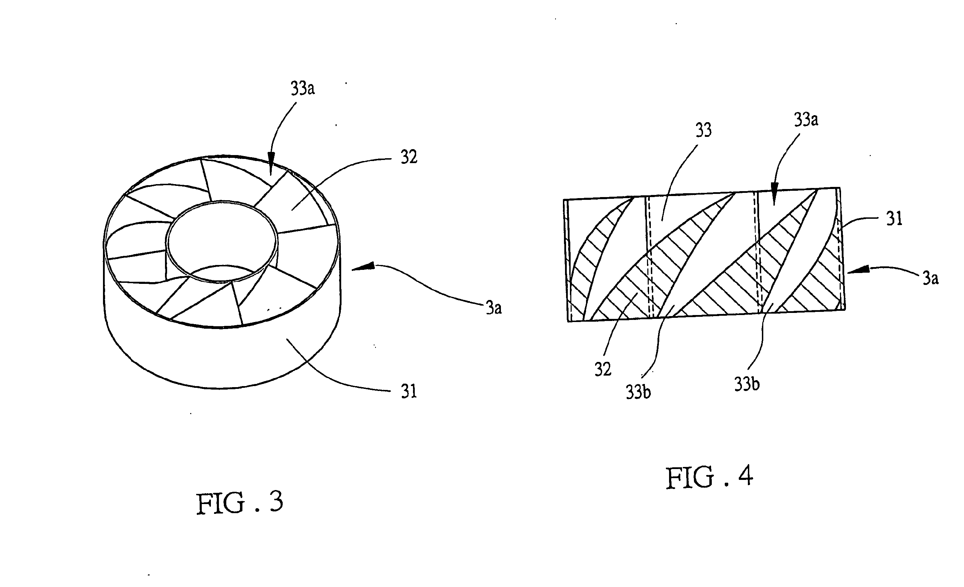

A further embodiment of the present invention can be designed to a diversion mechanism 3a has a plurality of spiral blades 32 on a ring unit 31, between two spiral blades 32 is a formation of a diversion channel 33, a fluid inlet hole 33a of the diversion channel 33 being larger than a fluid outlet hole 33b thereof. The inclination angle of the spiral blade 32 ranging from 91 to 179 degrees serves to adjust an angle of projected fluid flow according to the different length of the tube unit. As shown in FIGS. 3 and 4, the fluid flows from the fluid inlet tube 11 into the fluid-pressurizing chamber 13, then enters the fluid inlet hole 33a from a larger opening of the diversion channel 33, and flows out of the fluid outlet hole 33b from a smaller opening below, so the fluid can form an accelerated whirlpool in the tube unit 1, and the fluid being processed can be properly blended and achieve the better sterilizing effects as foresaid embodiment.

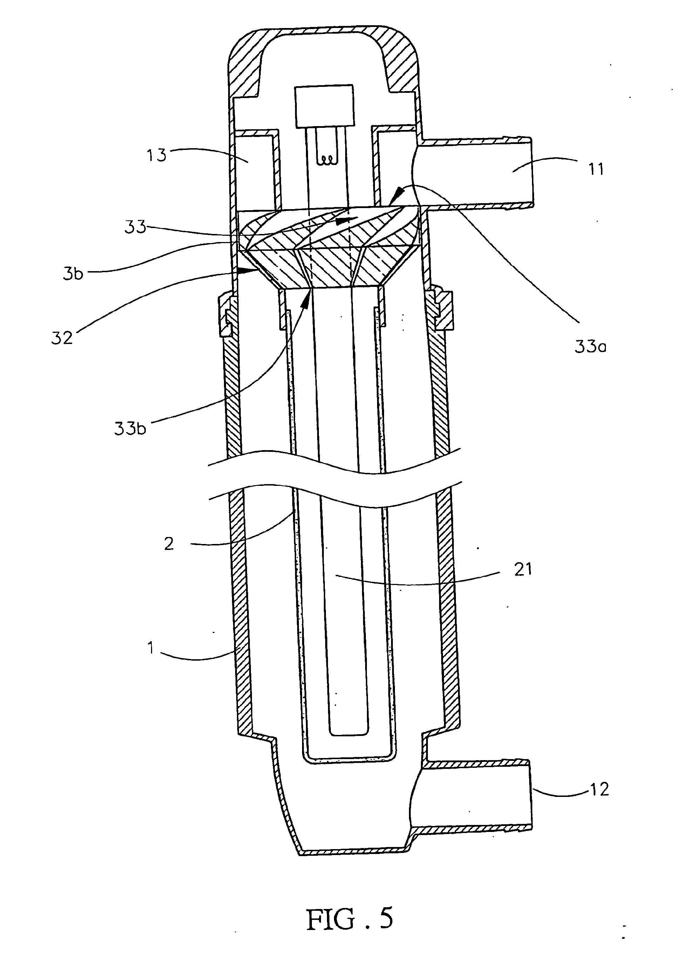

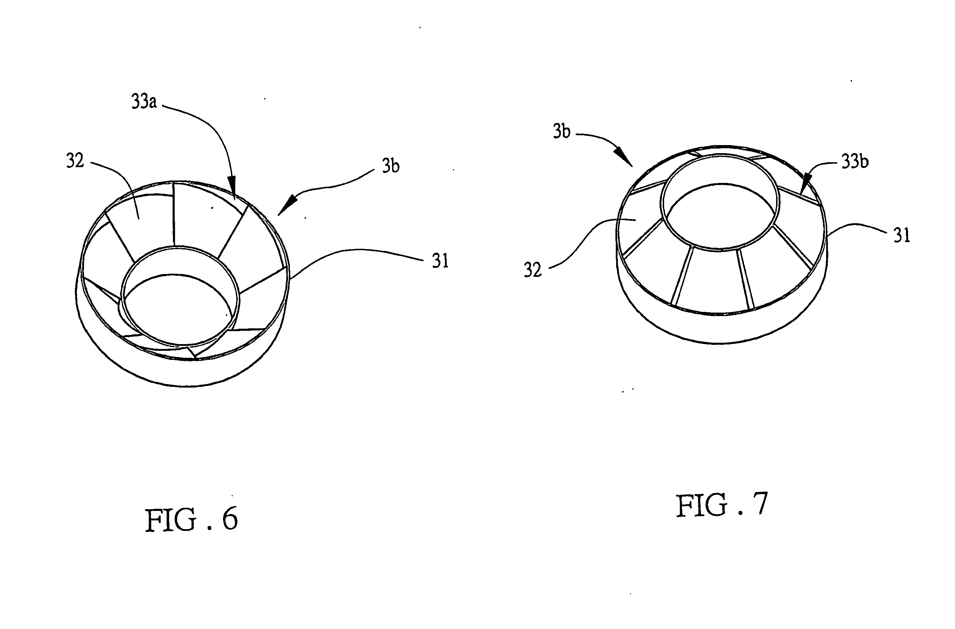

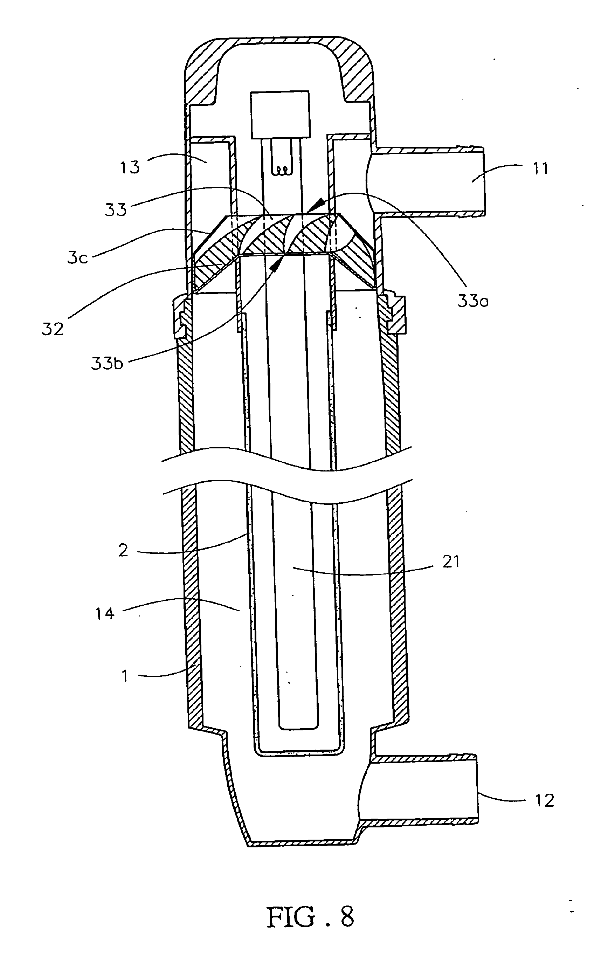

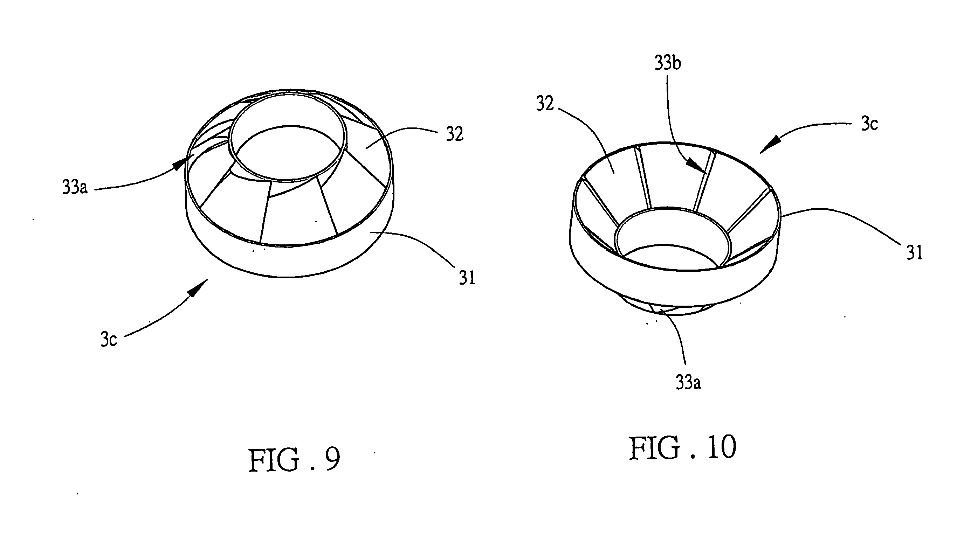

At a lower part of the diversion mechanism 3b is a tapered depression, as shown in FIGS. 5, 6 and 7, so the fluid outlet hole 33b between two adjacent blades 32 is facing toward the inner wall of the tube unit 1; or, at a lower part of the diversion mechanism 3c is a tapered protrusion, as shown in FIGS. 8, 9 and 10, so the fluid outlet hole 33b is facing toward the outer wall of the quartz sleeve 2. By the inclined direction of the fluid outlet hole 33b, the fluid flows through the diversion mechanism 3b or 3c, spins at high speed thus flushing the inner wall of the tube unit 1 and the outer wall of the quartz sleeve 2 to avoid dirt accumulation. According to the gravity, viscosity or flow rate of the fluid as well as the length or cubic measurements of the tube unit 1 and the inclination angle of the spiral blade 32, the upward or downward inclination angle of the diversion mechanism 3b or 3c can be adjusted from 1 to 89 degrees.

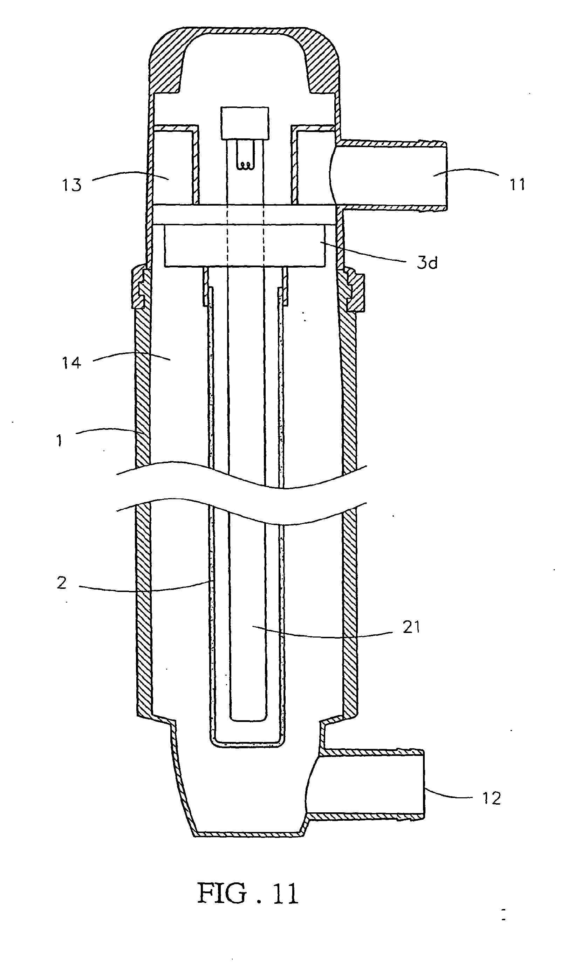

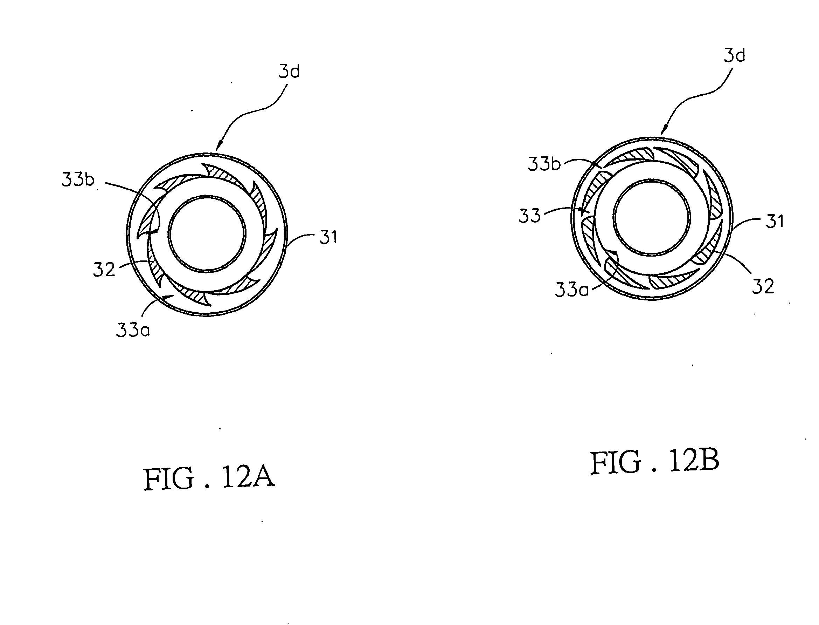

Please refer to FIG. 11, FIG. 12A & 12B, the diversion mechanism 3d can be designed to suit actual requirements to have a plurality of radial blades 32 on a ring unit 31. A diversion channel 33 is formed between every two adjacent radial blades 32. As shown in FIG. 12A, the fluid inlet hole 33a on the outside rim of the diversion channel 33 is larger than the fluid outlet hole 33b on the inside rim of the diversion channel 33, so the fluid flows through the fluid inlet tube 11, enters the fluid inlet hole 33a at the larger end on the outside of the diversion channel 33, and flows out of the fluid outlet hole 33b at the smaller end on the inside, enabling the fluid flow to become a rapidly spinning whirlpool inside the tube unit 1. Furthermore, the diversion mechanism 3d may be designed as required to have a plurality of radial blades 32 on a ring unit 31, between each two radial blades 32 being a formation of a diversion channel 33. The fluid inlet hole 33a on the inside rim is larger than the fluid outlet hole 33b on the outside rim of the diversion channel 33 (as shown in FIG. 12B), so the fluid can flow from inside to outside, creating a high-pressure whirlpool to achieve the above effects.

It should be noted that the above description and accompanying drawings are only used to illustrate some embodiments of the present invention and are not intended to limit the scope thereof. Any modification of the embodiments should fall within the scope of the present invention.

## Claims

1. An ultraviolet-radiation sterilizing fluid treatment device comprising a
quartz sleeve installed inside a tube unit, an ultraviolet-radiation lamp being located inside the quartz sleeve, a fluid inlet tube and a fluid outlet tube being located at the upper end and the lower end of the tube unit respectively;
**characterized by**:
a diversion mechanism disposed under the fluid inlet tube; said diversion mechanism having a plurality of diversion channels arranged in a ring unit while the total area of the fluid outlet holes of the diversion channels is smaller than the cross-sectional area of the passage of said tube unit containing the quartz sleeve so as to form a fluid-pressurizing chamber on top of this tube unit, said diversion mechanism having a plurality of spiral blades disposed in said ring unit whereby a diversion channel is formed between two adjacent spiral blades, whereby the fluid inlet hole of the diversion channel is larger than the fluid outlet hole of the diversion channel.

2. The ultraviolet-radiation sterilizing fluid treatment device as claimed in claim 1, wherein the spiral blade has an inclination angle in the range from 91 to 179 degrees.

3. The ultraviolet-radiation sterilizing fluid treatment device as claimed in claim 1 or 2, wherein at a lower part of the diversion mechanism is a tapered depression, the angle of upward inclination of the tapered depression ranging from 1 to 89 degrees.

4. The ultraviolet-radiation sterilizing fluid treatment device as claimed in claim 1 or 2, wherein at a lower part of the diversion mechanism is a tapered protrusion, the angle of downward inclination of the taper ranging from 1 to 89 degrees.

5. The ultraviolet-radiation sterilizing fluid treatment device as claimed in claim 1, 2, 3 or 4, wherein the fluid inlet holes of the diversion channels are located on an outside rim of the diversion channels and the fluid outlet holes of the diversion channels are located on an inside rim of the diversion channels.

6. The ultraviolet-radiation sterilizing fluid treatment device as claimed in claim 1, 2, 3 or 4, wherein the fluid inlet holes of the diversion channels are located on an inside rim of the diversion channels, and the fluid outlet holes of the diversion channels are located on an outside rim of the diversion channels.

## Patentansprüche

1. Behandlungsvorrichtung für Ultraviolettstrahlungs-Sterilisierungsfluid, umfassend eine
in einer Rohreinheit installierte Quarzbuchse, eine in der Quarzbuchse untergebrachte Ultraviolett-Strahlungslampe, ein Fluideinlassrohr und ein Fluidauslassrohr am oberen Ende bzw. am unteren Ende der Rohreinheit;
**gekennzeichnet durch**:
einen unterhalb des Fluideinlassrohrs angeordneten Ablenkungsmechanismus, wobei der Ablenkungsmechanismus eine Mehrzahl von Ablenkungskanälen aufweist, die in einer Ringeinheit angeordnet sind, während die Gesamtfläche der Fluidauslasslöcher der Ablenkungskanäle kleiner ist als die Querschnittfläche des Durchgangs der Rohreinheit, welche die Quarzbuchse enthält, um an der Oberseite dieser Rohreinheit eine Fluid-Druckkammer auszubilden, wobei der Ablenkungsmechanismus eine Mehrzahl von Spiralklingen besitzt, die in der Ringeinheit untergebracht sind, wobei zwischen zwei benachbarten Spiralklingen ein Ablenkungskanal ausgebildet ist und wobei das Fluideinlassloch des Ablenkungskanals größer ist als das Fluidauslassloch des Ablenkkanals.

2. Behandlungsvorrichtung für Ultraviolettstrahlungs-Sterilisierungsfluid gemäß Anspruch 1, wobei die Spiralklinge einen Neigungswinkel in der Größenordnung von 91 bis 179 Grad aufweist.

3. Behandlungsvorrichtung für Ultraviolettstrahlungs-Sterilisierungsfluid gemäß Anspruch 1 oder 2, wobei sich an einem unteren Teil des Ablenkungsmechanismus eine konische Vertiefung befindet, deren Winkel der Aufwärtsneigung von 1 bis 89 Grad reicht.

4. Behandlungsvorrichtung für Ultraviolettstrahlungs-Sterilisierungsfluid gemäß Anspruch 1 oder 2, wobei sich in einem unteren Teil des Ablenkungsmechanismus ein konischer Vorsprung befindet und der Winkel der Abwärtsneigung des Konus von 1 bis 89 Grad reicht.

5. Behandlungsvorrichtung für Ultraviolettstrahlungs-Sterilisierungsfluid gemäß Anspruch 1, 2, 3 oder 4, wobei die Fluideinlasslöcher der Ablenkungskanäle an einem Außenrand der Ablenkungskanäle angebracht sind und die Fluidauslasslöcher der Ablenkungskanäle an einem Innenrand der Ablenkungskanäle angeordnet sind.

6. Behandlungsvorrichtung für Ultraviolettstrahlungs-Sterilisierungsfluid gemäß Anspruch 1, 2, 3 oder 4, wobei die Fluideinlasslöcher der Ablenkungskanäle an einem Innenrand der Ablenkungskanäle angeordnet sind und die Fluidauslasslöcher der Ablenkungskanäle an einem Außenrand der Ablenkungskanäle angeordnet sind.

## Revendications

1. Dispositif de traitement de liquide par stérilisation aux ultraviolets, comprenant un manchon en quartz installé à l'intérieur d'une unité de tube, une lampe à ultraviolets disposée à l'intérieur du manchon en quartz, un tube d'entrée de liquide et un tube de sortie de liquide disposés, respectivement, à l'extrémité supérieure et à l'extrémité inférieure de l'unité de tube, **caractérisé en ce qu'**il comporte :
un mécanisme de dérivation disposé sous le tube d'entrée de liquide, lequel mécanisme de dérivation possède une pluralité de canaux de dérivation formant une unité annulaire tandis que l'aire totale des trous de sortie de liquide des canaux de dérivation est plus petite que l'aire de section du passage de ladite unité de tube contenant le manchon en quartz de façon à former une chambre de pressurisation du liquide par-dessus cette unité de tube, ledit mécanisme de dérivation possédant une pluralité d'aubes en spirale formant une unité annulaire, de sorte qu'un canal de dérivation est formé entre deux aubes en spirale adjacentes, le trou d'entrée de liquide du canal de dérivation étant plus grand que le trou de sortie de liquide du canal de dérivation.

2. Dispositif de traitement de liquide par stérilisation aux ultraviolets selon la revendication 1, **caractérisé en ce que** l'aube spirale a un angle d'inclinaison compris entre 91° et 179°.

3. Dispositif de traitement de liquide par stérilisation aux ultraviolets selon la revendication 1 ou 2, **caractérisé en ce qu'**il est prévu dans une partie inférieure du mécanisme de dérivation une dépression conique dont l'angle d'inclinaison vers le haut est compris entre 1° et 89°.

4. Dispositif de traitement de liquide par stérilisation aux ultraviolets selon la revendication 1 ou 2, **caractérisé en ce qu'**il est prévu dans une partie inférieure du mécanisme de dérivation une saillie conique dont l'angle d'inclinaison vers le bas est compris entre 1° et 89°.

5. Dispositif de traitement de liquide par stérilisation aux ultraviolets selon la revendication 1, 2, 3 ou 4, **caractérisé en ce que** les trous d'entrée de liquide des canaux de dérivation se trouvent sur un bord externe des canaux de dérivation et les trous de sortie de liquide des canaux de dérivation se trouvent sur un bord interne des canaux de dérivation.

6. Dispositif de traitement de liquide par stérilisation aux ultraviolets selon la revendication 1, 2, 3 ou 4, **caractérisé en ce que** les trous d'entrée de liquide des canaux de dérivation se trouvent sur un bord interne des canaux de dérivation, et les trous de sortie de liquide des canaux de dérivation se trouvent sur un bord externe des canaux de dérivation.
